# EUROPEAN PATENT SPECIFICATION

(11) **EP 1 569 578 B1**
(45) Date of publication and mention of the grant of the patent: **14.11.2007**
(21) Application number: 03773355.7
(22) Date of filing: 17.11.2003
(51) Int. Cl.: A61F 2/86, A61L 27/36

(54) **ENDOPROSTHESIS AND PROCESS TO OBTAIN**
ENDOPROTHESE UND HERSTELLUNGSVERFAHREN
ENDOPROTHESE ET PROCEDE DE FABRICATION CORRESPONDANT

(30) Priority: 21.11.2002 BR PI0205047; 27.06.2003 BR 0205047
(43) Date of publication of application: 07.09.2005
(73) Proprietor: Bueno, Ronaldo da Rocha Loures, Curitiba - Parana (BR)
(72) Inventor: Bueno, Ronaldo da Rocha Loures, Curitiba - Parana (BR)
(74) Representative: Winkler, Andreas Fritz Ernst
(86) International application number: PCT/BR2003/000168
(87) International publication number: WO 2004/045458

(56) References cited:
- EP-A2- 0 186 495
- EP-A2- 0 396 344
- EP-A2- 0 466 105
- WO-A1-00/33768

## Description

### FIELD OF THE INVENTION

The present invention relates to an endoprosthesis covered with biosynthetic cellulosic membrane (ECBCM), consisting of a prosthesis that comprises wire mesh structures covered with biocompatible material and, particularly, to a substantially cylindrical expandable endoprosthesis for the treatment of arterial stenosis, as well as the methods (not part of the invention) used to mount and deploy it.

### DESCRIPTION OF THE PRIOR ART

Coronary angioplasty, and arterial angioplasty in general, in human beings, is an effective procedure in reducing the severity of obstructive coronary artery disease.

Its long-term success is limited by the high rates of recurrence of the obstruction, with neointima proliferation (restenosis), that may affect up to 50% of certain subgroups of patients.

Several factors are involved in the restenosis mechanism, such as activation, migration and proliferation of smooth muscle cells of the arterial wall towards its lumen.

Among the resources used to reduce this percentage, the use of endoprosthesis, generally called stents, has reduced restenosis to nearly 20%.

Conventional stents are cylindrical meshes made of metal wires elastically deformable so as to be able to collapse while being introduced in the affected vessel.

After having been driven to the affected area by a catheter, using a balloon, the stent is released and expands against the inner walls of the artery, so as to stop the vessel from retracting.

Notwithstanding the success of stents in preventing restenosis, it still occurs in 15-20% of the patients. Restenosis may occur inside the stent, where treatment is difficult and restenosis very high, varying from 40% to 60%.

The intra-stent restenosis mechanism is entirely caused by intimal hyperplasia, that is, proliferation and subsequent migration through the mesh interstices, towards their lumen, of the arterial wall smooth muscle cells. Intra-stent restenosis treatment is difficult, and all available devices are ineffective in reducing its incidence.

Relative success has been obtained by administering systemic drugs to inhibit said proliferation. However, the use of systemic doses of medication when only a very small part of the vascular tree requires treatment is not a highly recommended procedure. Patients should not be exposed to the collateral effects of selective medications unnecessarily, and even the potential value of the therapeutic option may not be tolerated due to the risk that a systemic administration would cause.

Another problem associated with the use of conventional stents is the fact that they cannot prevent endothelium fragments resulting from the compression of the stent against the vessel wall to be released into the blood stream. These fragments act as microemboli that obstruct capillary vessels causing microinfarcts of the tissues they irrigate.

EP-A-396 344 relates to a hollow microbial cellulose comprising a cellulose produced by a microorganism. The microbial cellulose may be in the form of a composite comprising the microbial cellulose which is either integrated with another substance or adheres mainly to one or both of the inner and outer surface of a hollow carrier. The microbial cellulose is prepared by culturing a cellulose-producing microorganism on one or both of the inner surface and outer surface of an oxygen-permeable hollow carrier; or impregnating a cellulose produced by a microorganism with a medium, curing the cellulose if necessary, and cutting the cellulose.

### OBJECTS OF THE INVENTION

In view of the above, the first object of the invention is to provide a process to obtain a stent furnished with means to prevent or drastically reduce restenosis.

A second object consists in providing a device that is biocompatible with human tissues.

A further object of the invention is to provide a device furnished with means to administer medications locally.

Another object (not part of the invention) consists in providing a device that prevents endothelium fragments resulting from the compression of the stent against the vessel wall to be released into the blood stream.

Yet another object (not part of the invention) is to provide a method to mount the stent on an expandable balloon and deploy it in tube-shaped structures of the human body, such as blood vessels, among others.

Other objects consist in providing an ECBCM that renders proper anchorage for the cellulosic membrane, adequate fixation of the device to the vessel wall where it is installed, and that reduces local reactions resulting from the presence of a foreign body in contact with the vessel wall.

### SUMMARY OF THE INVENTION

The objects above, as well as others, are attained by the invention (a) through a process that provides the production of a cellulosic membrane in the form of an uneven surface, (b) through the biosynthesis of the cellulose on the inner surface of a mold, resulting from the fermentation of a culture of microorganisms with which said mold is filled.

According to another feature of the invention, the biosynthetic cellulosic membrane is obtained from a culture of the bacteria Acetobacter xylinum, or another suitable cellulose-producing microorganism, in a liquid medium.

According to a further feature of the invention, said culture medium is placed in a mold made of a liquidproof material that is nevertheless permeable to gases.

According to another feature of the invention, the membrane is formed on the inner surface of such mold.

According to yet another feature of the invention, such membrane resembles in every detail the inner surface of said mold.

According to another feature of the invention, said membrane is in the shape of a tube that involves a cylindrical stainless steel wire mesh.

According to a further feature of the invention, said cellulosic membrane is impregnated with a therapeutic substance.

According to other features of the invention, means of anchorage are applied externally to the device.

According to such features, said means of anchorage consists of a second stent placed externally to the cellulosic membrane, compressing it against the first internal stent, in such a way as to form a three-layer structure, where the two outer layers are made up of the stainless steel wire meshes of the first and second stents and the inner layer is made up of the cellulosic membrane.

Alternately, such means consists in clinching the front and back borders of the first, inner, stent so as to incarcerate the corresponding borders of the cellulosic membrane.

In yet another solution, such means consists in applying two expandable rings made of inert biocompatible material, placed on the front and back ends of the cellulosic membrane, in such a way as to compress it against the corresponding ends of the first, inner, stent, during the introduction and maneuvering stages.

### DESCRIPTION OF THE DRAWINGS

Advantages and features of the invention will be better understood through the description of preferred carrying out modes and related drawings, where:
Figure 1 shows an uncovered stent, depicted in accordance with the known technique.
Figure 2 illustrates in a schematic perspective view the process to obtain the biosynthetic cellulosic membrane, pursuant to the principles of the invention.
Figure 3 illustrates in perspective an ECBCM in accordance with the principles of the invention.
Figure 4 illustrates an alternative construction of an ECBCM, made up of a first and a second stents arranged concentrically with the biosynthetic cellulosic membrane placed between them.
Figure 5 illustrates a form of fixation of the biosynthetic cellulosic membrane, by clinching the borders of the inner stent, in front view and partial cross-section.
Figure 6 illustrates a form of fixation of the biosynthetic cellulosic membrane, by the use of expandable rings.

### DESCRIPTION OF THE PREFERRED EMBODIMENT

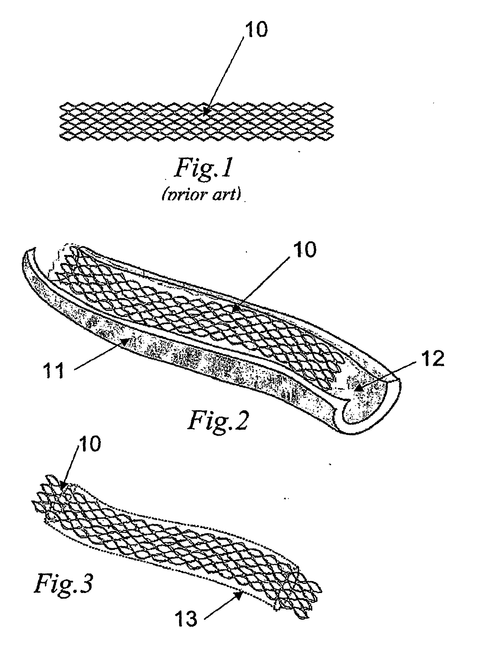

A closer look at Figure 1 will reveal the traditional stent consisting of a substantially cylindrical body (10) with walls made of a wire mesh of stainless steel, preferably 316LVM, or any other metal with biocompatible features. The inner surface is polished so as to render it as smooth as possible, in order to avoid the adherence of fibrin particles, plaques, etc. The outer surface, on the contrary, is rough so as to promote a better anchorage of the biosynthetic cellulosic membrane.

The process used to obtain the membrane is shown schematically in Figure 2. As the figure shows, a stainless steel stent (10) is inserted in a tubular mold (11) of a slightly larger diameter filled with a culture medium inoculated with the bacteria Acetobacter xylinum. The culture medium used presents the following composition:
Peptone 5.0 g/l
Yeast extracts 5.0 g/l
Na2HP04 2.7 g/l
Citric acid 1.15 g/l
Glucose 20.0 g/l

The invention is based on the fact that the material used in making the mold is impermeable to liquids but permeable to gases. This property can be found in several polymers such as silicon and Teflon. In this case, silicon is used to make the mold. As demonstrated by Borzani and Souza *(*Borzani, W e Souza, S.J. - "Mechanism of Film Thickness Increase During the Production of Cellulose on Non-Agitated Liquid Media" - Biotechnology Letters, 1995, vol. 17, pp. 1271-1272), it is in the liquid/air interface that the biosynthetic cellulosic membrane is formed. Using a material that is permeable to gases will put the liquid surface that is in contact with the mold wall in contact with the gases in the atmosphere. Consequently, it will be formed *in loco,* by biosynthesis, a cellulosic membrane juxtaposed to the inner wall of the mold, as a perfect reproduction of said wall.

The width of said membrane will depend on conditions such as temperature - kept between 15 and 32°C (degrees centigrade)- and the time of fermentation, which varies from 48 to 240 hours.

Once the time is up for the membrane to be formed involving the stent, the ends of the silicon tube are opened and the culture medium and the cellulosic tubular membrane, as well as the stent covered with it, are removed.

After the cellulose-covered stent is removed from the silicon mold, it is submitted to a chemical treatment so as to free it from proteins and cells and other elements resulting from bacterial activity.

Typically, this treatment comprises immersion in sodium lauryl sulfate at 0.5-5% for a period that may vary from 2 to 24 hours. The covered stent is then rinsed by agitation with distilled water, changed 5 to 10 times, until the sodium lauryl sulfate residues are totally eliminated. It is then treated with a sodium hydroxide solution at 0.5-5% for 2 to 24 hours. After that the sodium hydroxide solution is neutralized by rinsing the covered stent with distilled water, changed 5 to 15 times. Controlling the rinse water Ph will guarantee the procedure.

After the chemical treatment is completed, the covered stent goes through a drying process, in a drying chamber with filtered air so as to prevent contamination and the presence of solid particles in suspension in the air. During the drying process, there is a retraction of the cellulosic fibers of the membrane that covers the stent, which results in better adjustment and adherence of the membrane to the structure of the stent.

As already described, restenosis is due to the migration of neointima tissue through the stent interstices towards the arterial lumen. The biosynthetic cellulose membrane, involving the stent externally, sets up a barrier to the migration of the smooth muscle cells of the artery medium layer towards the arterial lumen.

Because it is totally biocompatible, the endoprosthesis neo-endothelization will be extremely fast, thus reducing the risk of subacute thrombosis. Thus, both the vessel wall and the blood elements will be in contact with a material that is 100% biocompatible, enabling its normal flow.

Another advantage of the cover obtained by the process described consists in the fact that it provides a vehicle for the topic application of post-angioplasty restenosis inhibiting drugs, so that the medicine is available for release during a few weeks. Till these days, the most consistent and encouraging results were obtained with bare wire (not involved with any kind of membrane) stents impregnated with rapamycin, an immunosuppressive anti-proliferating agent.

Figure 3 illustrates the stent covered with the biosynthetic cellulosic membrane 13 (ECBCM) according to the invention.

As already mentioned, when conventional stents are compressed against the vessel wall, they may macerate the endothelium and release endothelium fragments into the blood stream. The cellulosic membrane-covered stent, due to its very configuration, retains these fragments in place, and they are not released into the blood stream because they are incarcerated between the vessel wall and the cellulosic membrane.

An alternate covered stent, of a more immediate application and lower cost, may be obtained by the inclusion of a step when the cellulose-stent ensemble is rehydrated before it is crimped on the balloon, as will be described herein, upon presentation of the method (not part of the invention) used to mount and release the stent.

This alternative applies the methodology already described to readymade stents, available in the marketplace.

Since during the drying process there is a slight natural tendency of the cellulose to invaginate between the stent wires towards the lumen (to the inside) as if there was a suction from the inside of the stent-cellulose ensemble lumen, when the cellulose-stent prototype is rehydrated and crimped on the balloon, the cellulose accommodates between the stent wires, as if it had a memory, filling the spaces between the wires (as when an umbrella is closed).

The cellulose is thus tightly held between the stent wires in a rather safe manner.

It is possible, then, to make in laboratory a safe prototype featured with a firm membrane that will not move because it is compressed between the wires of a single stent, taking advantage of its low profile, its flexibility, its architecture, etc.

The cellulose must be firmly fastened to the stent because one has to make sure that the stent-cellulose ensemble (upon its introduction into the blood circulation, up to the place where it is released) will guarantee that the cellulose won't be detached from the stent on the way to deployment.

Clearly the use of the stents described in this patent is not limited to the vascular system, but can be extended to any and all tubular structures in the human body, such as the digestive tube, trachea, bronchi, bile ducts, urethra, ureter and Fallopian tubes.

It is also understood that the modes of carrying out described herein are mere exemplifiers of the invention, which admits therefore other variations and modifications within the grasp of any technician, provided they don't surpasses the conceptual limits of the invention.

Although the example used bacteria of the genus Acetobacter subspecies xylinum for the production of cellulose, other bacteria, such as Acetobacter pasteurianus, Acetobacter rancens, Bacterium xylinoides, may be employed, for they are also capable of producing cellulose.

Thus, according to one aspect of the invention, in the process used to obtain ECBCM, the risks resulting from restenosis after the stent is deployed are eliminated by covering such stent with a tubular membrane of biosynthetic cellulose that may be powered by the release of local anti-proliferating drugs.

According to another aspect of the invention, in the process used to obtain ECBCM, the cover is obtained through the fermentation of a culture medium inoculated with cellulose-producing bacteria inside a mold made of material that is impermeable to liquids and permeable to gases.

According to yet another aspect of the invention, in the process used to obtain ECBCM, the material of the mold is silicon.

According to a further aspect of the invention, in the process used to obtain ECBCM, the mold is tube shaped with a diameter slightly larger than that of the metal wire stent inserted in it.

According to another aspect of the invention, in the ECBCM, the spaces between the wires that form the stent mesh are totally closed by the biosynthetic cellulosic membrane that covers such mesh.

According to yet another aspect of the invention, with the ECBCM, the biosynthetic cellulosic membrane that involves the stent blocks the migration of smooth muscles cells towards the artery lumen after angioplasty.

According to another additional aspect of the invention, with the ECBCM, the release of endothelium fragments (that are a result of the stent being compressed against the vessel wall) into the blood stream is hampered because such fragments are incarcerated between the vessel wall and the cellulosic membrane.

According to yet another additional aspect of the invention, with the ECBCM, local administration of therapeutic substances is provided by impregnating such cellulosic membrane with said substances.

According to one more additional aspect, with the method used to mount and deploy the ECBCM, it is rehydrated and mounted through its compression on an expandable balloon, of the type that is routinely used in inside procedures.

The expandable balloon, deflated together with the compressed ECBCM, significantly reduces the diameter of the ensemble that may be reduced to one millimeter, for a 3.0 mm coronary, for example.

With this resulting diameter, the ECBCM can navigate from the great circulation (aorta) to vessels and tubular structures as small as 1.5 mm in diameter.

Once it gets to the desired point of deployment in the human body, the balloon against which the ECBCM is compressed is inflated, and this procedure will release and compress the endoprosthesis against the vessel wall or any other tubular structure in the body.

After this procedure is completed, the expandable balloon is deflated and withdrawn from the body, and the ECBCM remains firmly in place.

According to another alternative aspect of the invention, the ECBCM may be self-expanding, dispensing with the balloon for its release on the inside of the vessel. This is possible if one uses a metal with a memory of its previously conceived diameter, which can be compressed and is able to go back to its predefined diameter (as with a spring).

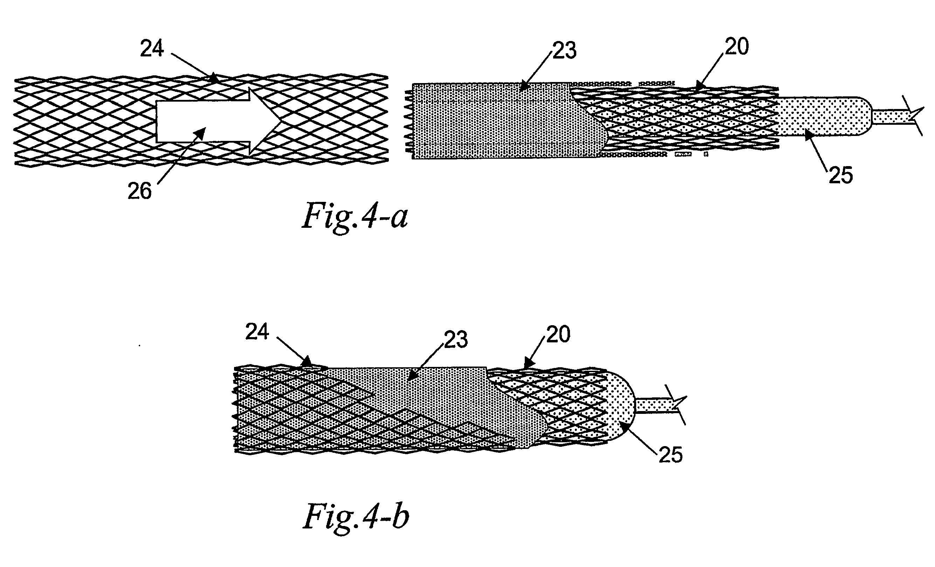

With reference to Figure 4, it is now presented a first carrying out of the ECBCM alternative, with two stents, where a second mesh (24) substantially concentric with the first mesh (20) involves the ensemble formed by the cellulosic membrane (23) and said first mesh.

To make this device, the ensemble formed by the first mesh (20), the cellulose membrane (23) and the angioplasty balloon (25) is inserted into the second mesh (24), the diameter of which must be larger than that of the first mesh, as illustrated in Figure 4-a.

The balloon (25) is then insufflated so as to expand the mesh (20) (first stent) and the cellulosic membrane until it is juxtaposed internally to the second mesh (24) (second stent)-, as shown in Figure 4-b. This operation causes the cellulosic membrane to be held by the mutual compression of both stents, so as not to move while the device navigates through the vascular system.

For the deployment of the proposed device, a deflated angioplasty balloon is introduced inside the ensemble and this is compressed from without so that both stents - with the cellulosic membrane imprisoned between them - are reduced in diameter. At this step of the process, the external diameter of the ensemble is less than 1.0 mm, so as to allow for safe maneuvering inside the blood vessels.

When it reaches the point of deployment, the angioplasty balloon is insufflated with a pressure of 8 atmospheres, causing the expansion of the inner and the outer stents as well as the cellulosic membrane. The balloon is then deflated and withdrawn. In this case, the radial force exerted on the vessel walls result from the addition of the individual forces of the first (inner) stent (20) and of the second (outer) stent (24), each corresponding substantially to 50% of the total force.

Since said total force results from the sum of the individual expansion forces of the two elastic structures (20 and 24), it is possible to use thinner stents as compared to the device that comprises only a single stent (20). In numerical terms, as regards a single stent, it was experimentally determined that in order to resist the artery contracting force, it would have to be 12 to 16% metal, whereas the empty spaces between the wires would represent 84 - 88 % of the stent. Furthermore, this quantity of metal doesn't cause any significant reaction (foreign body). When a two-stent structure is adopted, the metal area in each stent can be reduced to something around 7 - 8 %, after the expansion. There are two advantages to this: first, since the cellulosic membrane isolates the inner stent from the outer stent, as a result there is less metal in contact with the circulation, and therefore the possibility of a reaction to a foreign body is decreased. Second, there is less metal in contact with the vessel wall, reducing the possibility of reaction in this site also. An additional advantage results from the fact that the structure formed by two meshes (8% metal plus 8% metal) is more flexible than one single 16% metal mesh, making it easier to maneuver in tortuous vessels.

The alternate structure proposed, comprising a cellulosic membrane interposed between two meshes of inert material - such as stainless steel - presents a better performance after deployment than its PTFE similar, because the biocompatibility of the cellulose stimulates a faster neo-endothelization as compared with PTFE structures.

It should be noted that, although the initial description was based on a stainless steel wire mesh, the very same result might be obtained if the stent were a laser tooled thin wall tube.

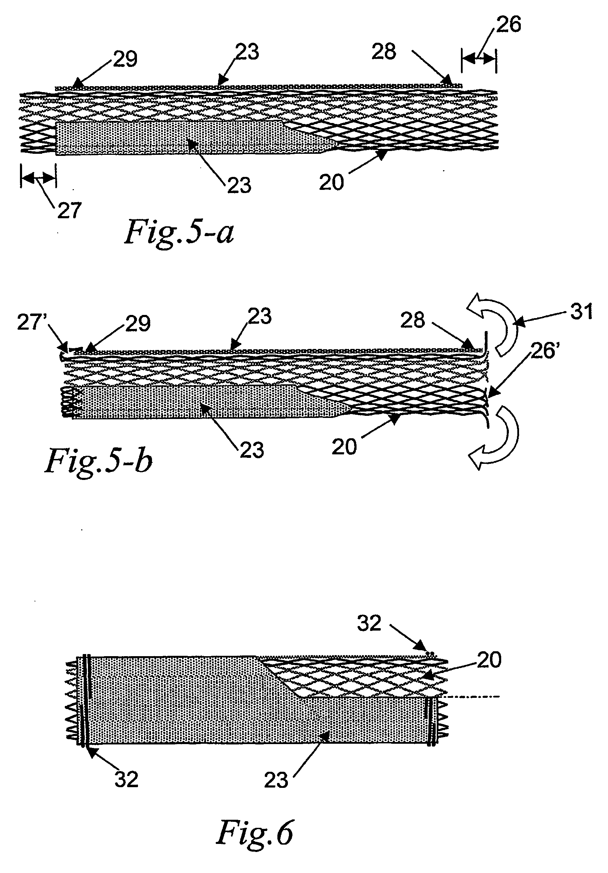

Another mode of carrying out this alternative is shown in Figure 5. In this case, the stent mesh (20) has extensions (26 and 27) in both extremities that stretch beyond the ends (28 and 29) of the cellulosic membrane (23), as seen in Figure 5-a. These extensions are then expanded outward, as with (26') in Figure 5-b, and clinched as the arrows (31) indicate. As a result of this operation, annular channels are formed encircling both ends of the stent mesh, as exemplified by channel (27'). Note that this channel involves the end (29) of the cellulosic membrane (23) imprisoning it and preventing it from sliding along the stent while it is introduced into the vascular system.

A second alternative manner of providing the retention of said membrane (23) consists in providing the radial constriction of its extremities against the inner stent (20) using external expansile rings (32), as shown in Figure 6. These rings should be set in such a way as to compress the membrane when the ensemble is collapsed for introduction into the vascular system, expanding together with the stent when the fixation occurs by insufflation of the angioplasty balloon.

According to all aspects above, the invention is defined and delimited by the following claims.

## Claims

1. **PROCESS TO OBTAIN ENDOPROSTHESIS** wherein the endoprosthesis is covered with biosynthetic cellulosic membrane (13) shaped as an uneven membrane that is produced *in loco* by a culture of microorganisms that synthesize cellulose.

2. **PROCESS TO OBTAIN ENDOPROSTHESIS** according to claim 1 wherein the membrane (13) is shaped by using a mold (11) made of a material that is impermeable to liquids and permeable to gases, and that said mold (11) is filled with a culture medium (12) inoculated with cellulose-producing bacteria.

3. **PROCESS TO OBTAIN ENDOPROSTHESIS** according to claim 2 comprising the steps:
A wire mesh structure (10), with a shape that is substantially equal to the shape of the endoprosthesis, is introduced into the mold (11);
The mold (11) is filled with a culture medium (12) that was previously inoculated with biosynthetic cellulose-producing bacteria;
The mold (11) is closed and the cellulosic membrane (13) is formed by fermentation under controlled temperature for a certain period of time;
The ensemble formed by the cellulosic membrane-covered mesh is removed, and treated with a sodium hydroxide solution followed by rinsing procedures; treated with a sodium lauryl sulfate solution followed by rinsing procedures.
The ensemble is dried using filtered air.

4. **PROCESS TO OBTAIN ENDOPROSTHESIS** according to claim 3 wherein the material in said mold (11) is silicon.

5. PROCESS TO OBTAIN ENDOPROSTHESIS according to claim 3 wherein the temperature is kept between 3 and 40°C (degrees Centigrade) while the membrane (13) is being formed by fermentation.

6. **PROCESS TO OBTAIN ENDOPROSTHESIS** according to claim 3 wherein the time it takes for the membrane (13) to form by fermentation varies from 16 to 240 hours.

7. **PROCESS TO OBTAIN ENDOPROSTHESIS** according to claim 3 wherein the ensemble is treated with a sodium lauryl sulfate solution at a concentration between 0.5% and 5 % for a period of 2 to 24 hours;

8. **PROCESS TO OBTAIN ENDOPROSTHESIS** according to claim 3 wherein the ensemble is treated with a sodium hydroxide solution at a concentration between 0.5% and 5 % for a period of 2 to 24 hours;

9. **PROCESS TO OBTAIN ENDOPROSTHESIS** according to claim 2 or 3 wherein the cellulose-producing bacteria are bacteria of the group that comprises the genus Acetobacter subspecies xylinum, pasteurianus, rancens, as well as Bacterium xylinoides.

10. **PROCESS TO OBTAIN ENDOPROSTHESIS** according to claim 9 wherein the culture medium (12) is inoculated with the bacteria Acetobacter xylinum.

11. **PROCESS TO OBTAIN ENDOPROSTHESIS** according to claim 9 or 10 wherein the culture medium (12) is adequate to the production of biosynthesized cellulose.

12. **ENDOPROSTHESIS** wherein it is covered with biosynthetic cellulosic membrane (13) made up of a wire mesh structure (10), with a shape that is substantially equal to the shape of the endoprosthesis, externally covered with a biosynthetic cellulosic membrane (13).

13. **ENDOPROSTHESIS** according to claim 12, wherein said structure (10) consists of a stainless steel wire mesh body that is substantially cylindrical in shape, and is externally covered with a substantially cylindrical membrane (13) of biosynthetic cellulose.

14. **ENDOPROSTHESIS** according to claim 13, wherein said structure features a highly polished inner face and a rough outer surface.

15. **ENDOPROSTHESIS** according to claims 12, 13 or 14, wherein said membrane (13) is impregnated with a therapeutic substance.

16. **ENDOPROSTHESIS** according to claim 12, wherein said structure is made up of self-expandable metal with memory of its preconceived diameter.

17. **ENDOPROSTHESIS** according to claims 12-14, wherein there is a provision of external means of retention (24, 26', 27', 32) to keep such membrane (23) in place.

18. **ENDOPROSTHESIS** according to claim 17, wherein such external means of retention consists in a second expandable mesh substantially cylindrical in shape (24) made of a flexible inert material that is juxtaposed to such membrane (23).

19. **ENDOPROSTHESIS** according to claim 18, wherein the proportion of material as related to empty spaces in both the first (20) and the second (24) meshes, after expansion, is between 6% and 8%.

20. **ENDOPROSTHESIS** in accordance with claim 17, wherein said external means of retention consist of annular channels (27') at both ends of the stent mesh (20), involving and imprisoning the extremities. (28, 29) of the cellulosic membrane (23).

21. **ENDOPROSTHESIS** in accordance with claim 17, wherein such external means of retention result from a radial constriction provided by expandable rings (32) placed over both extremities of the cellulosic membrane (23).

22. **PROCESS TO OBTAIN ENDOPROSTHESIS** according to claim 1, wherein the endoprosthesis is covered with biosynthetic cellulosic membrane (23) that is made up of a first expandable mesh that is substantially cylindrical in shape (20) and is made of flexible inert material, covered externally by an uneven membrane (23) substantially cylindrical in shape, produced *in loco* by a culture of cellulose synthesizing microorganisms, including the steps:
The ensemble formed by such first structure (first stent) (20) and said membrane (23), with a deflated balloon (25) inside, is inserted in a second structure (second stent) (24) of a larger diameter than that of the first structure (20);
Said balloon (25) is insufflated and expands such first structure (first stent) (20) and the cellulosic membrane (23) until it is internally juxtaposed to said second structure (24) (second stent), providing the retention by compression of said cellulosic membrane (23).

23. **PROCESS TO OBTAIN ENDOPROSTHESIS** according to claim 1, wherein the endoprosthesis is covered with biosynthetic cellulosic membrane (23) that is made up of a first expandable mesh that is substantially cylindrical in shape (20) and is made of flexible inert material, covered externally by an uneven membrane (23) substantially cylindrical in shape, produced *in loco* by a culture of cellulose synthesizing microorganisms, including the step of clinching (31) of the extremities (26, 27) of said mesh that stretch beyond the ends (28,29) of said cellulosic membrane (23), forming annular channels (27') that encircle both extremities of said mesh and enclose the ends (28, 29) of said membranes (23).

## Patentansprüche

1. Verfahren zur Herstellung einer Endoprothese, wobei die Endoprothese mit biosynthetischer Cellulosemembran (13) bedeckt wird, die als eine unebene Membran (13) geformt ist, die an Ort und Stelle durch eine Kultur von Mikroorganismen, die Cellulose synthetisieren, hergestellt wird.

2. Verfahren zur Herstellung einer Endoprothese nach Anspruch 1, wobei die Membran (13) durch Verwendung einer Form (11) geformt wird, die aus einem Material hergestellt ist, das gegenüber Flüssigkeiten impermeabel und gegenüber Gasen permeabel ist, und wobei die Form (11) mit einem Kulturmedium (12) gefüllt wird, das mit Cellulose erzeugenden Bakterien beimpft ist.

3. Verfahren zur Herstellung einer Endoprothese nach Anspruch 2, welches die Schritte umfaßt:
eine Drahtnetzstruktur (10) mit einer Form, die im wesentlichen gleich ist zu der Form der Endoprothese, wird in die Form (11) eingeführt;
die Form (11) wird mit einem Kulturmedium (12) befüllt, das zuvor mit biosynthetischen, Cellulose erzeugenden Bakterien beimpft worden ist;
die Form (11) wird verschlossen und die Cellulosemembran (13) durch Fermentation unter gesteuerter Temperatur für eine bestimmte Zeitdauer gebildet;
der durch das mit einer Cellulosemembran bedeckte Netz gebildete Aufbau wird entfernt und mit einer Natriumhydroxidlösung behandelt, gefolgt von Spülarbeitsabläufen; wird mit einer Natriumlaurylsulfatlösung behandelt, gefolgt von Spülarbeitsabläufen;
der Aufbau wird unter Verwendung filtrierter Luft getrocknet.

4. Verfahren zur Herstellung einer Endoprothese nach Anspruch 3, wobei das Material in der Form (11) Silicium ist.

5. Verfahren zur Herstellung einer Endoprothese nach Anspruch 3, wobei die Temperatur zwischen 3 und 40°C (Grad Celsius) gehalten wird, während die Membran (13) durch Fermentation gebildet wird.

6. Verfahren zur Herstellung einer Endoprothese nach Anspruch 3, wobei die Zeit, die es dauert, die Membran (13) durch Fermentation zu bilden, von 16 bis 240 Stunden variiert.

7. Verfahren zur Herstellung einer Endoprothese nach Anspruch 3, wobei der Aufbau mit einer Natriumlaurylsulfatlösung in einer Konzentration zwischen 0,5% und 5% für eine Dauer von 2 bis 24 Stunden behandelt wird.

8. Verfahren zur Herstellung einer Endoprothese nach Anspruch 3, wobei der Aufbau mit einer Natriumhydroxidlösung in einer Konzentration zwischen 0,5% und 5% für eine Dauer von 2 bis 24 Stunden behandelt wird.

9. Verfahren zur Herstellung einer Endoprothese nach Anspruch 2 oder 3, wobei die Cellulose erzeugenden Bakterien Bakterien aus der Gruppe sind, die die Gattung Acetobacter subspecies xylinum, pasteurianus, rancens sowie Bacterium xylinoides umfaßt.

10. Verfahren zur Herstellung einer Endoprothese nach Anspruch 9, wobei das Kulturmedium (12) mit dem Bakterium Acetobacter xylinum beimpft wird.

11. Verfahren zur Herstellung einer Endoprothese nach Anspruch 9 oder 10, wobei das Kulturmedium (12) adäquat zur Herstellung einer biosynthetisierten Cellulose ist.

12. Endoprothese, wobei sie bedeckt ist mit einer biosynthetischen Cellulosemembran (13), die aus einer Drahtnetzstruktur (10) mit einer Form, die im wesentlichen gleich ist zur Form der Endoprothese, aufgebaut ist, äußerlich bedeckt mit einer biosynthetischen Cellulosemembran (13).

13. Endoprothese nach Anspruch 12, wobei die Struktur (10) aus einem Edelstahldrahtnetzkörper besteht, der der Form nach im wesentlichen zylindrisch ist und äußerlich bedeckt ist mit einer im wesentlichen zylindrischen Membran (13) aus biosynthetischer Cellulose.

14. Endoprothese nach Anspruch 13, wobei die Struktur eine hochpolierte innere Fläche und eine rauhe äußere Fläche aufweist.

15. Endoprothese nach Ansprüchen 12, 13 oder 14, wobei die Membran (13) mit einer therapeutischen Substanz imprägniert ist.

16. Endoprothese nach Anspruch 12, wobei die Struktur aufgebaut ist aus sich selbst ausdehnbarem Metall mit Speicherfähigkeit ihres vorher vorhandenen Durchmessers.

17. Endoprothese nach Ansprüchen 12-14, wobei es eine Bereitstellung eines äußeren Haltemittels (24, 26', 27', 32) gibt, um eine solche Membran (23) an Ort und Stelle zu bewahren.

18. Endoprothese nach Anspruch 17, wobei ein solches äußeres Haltemittel in einem zweiten expandierbaren Netz besteht, das der Form nach im wesentlichen zylindrisch (24) ist und aus einem flexiblen inerten Material gemacht ist, das an eine solche Membran (23) angestellt ist.

19. Endoprothese nach Anspruch 18, wobei der Anteil an Material in bezug auf Leerräume in sowohl den ersten (20) als auch den zweiten (24) Netzen, nach der Expansion, zwischen 6% und 8% ist.

20. Endoprothese nach Anspruch 17, wobei das äußere Haltemittel aus ringförmigen Kanälen (27') an beiden Enden des Stentnetzes (20) besteht, involvierend und einschließend die Endbereiche (28, 29) der Cellulosemembran (23).

21. Endoprothese nach Anspruch 17, wobei ein solches äußeres Haltemittel aus einer radialen Konstriktion resultiert, bereitgestellt durch expandierbare Ringe (32), die über beiden Endbereiche der Cellulosemembran (23) angeordnet sind.

22. Verfahren zur Herstellung einer Endoprothese nach Anspruch 1, wobei die Endoprothese mit biosynthetischer Cellulosemembran (23) bedeckt wird, aus einem ersten expandierbaren Netz aufgebaut ist, das der Form nach im wesentlichen zylindrisch (20) ist und aus flexiblem inerten Material hergestellt ist, äußerlich bedeckt durch eine unebene Membran (23), die der Form nach im wesentlichen zylindrisch ist, hergestellt an Ort und Stelle durch eine Kultur von Cellulose synthetisierenden Mikroorganismen, welches die Schritte einschließt:
der Aufbau, der durch eine solche erste Struktur (erster Stent) (20) und die Membran (23) gebildet wird, mit einem entleerten Ballon (25) im inneren, wird in eine zweite Struktur (zweiter Stent) (24) eines größeren Durchmessers als derjenige der ersten Struktur (20) insertiert;
der Ballon (25) wird aufgeblasen und dehnt somit eine solche erste Struktur (erster Stent) (20) und die Cellulosemembran (23), bis sie intern an der zweiten Struktur (24) (zweiter Stent) anliegen, bereitstellend die Retention durch Kompression der Cellulosemembran (23).

23. Verfahren zur Herstellung einer Endoprothese nach Anspruch 1, wobei die Endoprothese mit biosynthetischer Cellulosemembran (23) bedeckt ist, hergestellt wird aus einem ersten expandierbaren Netz, das der Form nach im wesentlichen zylindrisch ist (20) und aus flexiblem inerten Material hergestellt ist, bedeckt äußerlich durch eine unebene Membran (23), die der Form nach im wesentlichen zylindrisch ist, hergestellt an Ort und Stelle durch eine Kultur von Cellulose synthetisierenden Mikroorganismen, einschließlich den Schritt eines Umklammerns (31) der Endbereiche (26, 27) des Netzes, das sich über die Enden (28, 29) der Cellulosemembran (23) streckt, ausbildend ringförmige Kanäle (27'), die beide Endbereiche des Netzes umschließen und die Enden (28, 29) der Membran (23) umhüllen.

## Revendications

1. Processus visant à obtenir une endoprothèse, dans lequel l'endoprothèse recouverte d'une membrane cellulosique biosynthétique (13) conçue sous la forme d'une membrane inégale qui est produite in loco par une culture de microorganismes qui synthétisent de la cellulose.

2. Processus visant à obtenir une endoprothèse selon la revendication 1, dans lequel la membrane (13) est formée à l'aide d'un moule (11) composé d'un matériau qui est imperméable aux liquides et perméables aux gaz, et dans lequel ledit moule (11) est rempli d'un milieu de culture (12) inoculé avec des bactéries produisant de la cellulose.

3. Processus visant à obtenir une endoprothèse selon la revendication 2 comprenant les étapes consistant à :
introduire dans le moule (11) une structure de treillis métallique (10) avec une forme qui est essentiellement égale à la forme de l'endoprothèse ;
remplir le moule (11) d'un milieu de culture (12) qui a été précédemment inoculé avec des bactéries produisant de la cellulose biosynthétique ;
fermer le moule (11) et former la membrane cellulosique (13) par fermentation sous une température contrôlée pour une certaine période de temps ;
retirer l'ensemble formé par la maille recouverte d'une membrane cellulosique et le traiter avec une solution d'hydroxyde de sodium suivi de procédures de rinçage, le traiter avec une solution de laurylsulfate de sodium suivi de procédures de rinçage.
L'ensemble est séché en utilisant de l'air filtré.

4. Processus visant à obtenir une endoprothèse selon la revendication 3, dans lequel le matériau dans ledit moule (11) est de la silicone.

5. Processus visant à obtenir une endoprothèse selon la revendication 3, dans lequel la température est maintenue entre 3 et 40°C (degrés centigrade) tandis que la membrane (13) est formée par fermentation.

6. Processus visant à obtenir une endoprothèse selon la revendication 3, dans lequel le temps nécessaire pour former la membrane (13) par fermentation varie entre 16 et 240 heures.

7. Processus visant à obtenir une endoprothèse selon la revendication 3, dans lequel l'ensemble est traité avec une solution de laurylsulfate de sodium à une concentration comprise entre 0,5 % et 5 % pendant une période de 2 à 24 heures.

8. Processus visant à obtenir une endoprothèse selon la revendication 3, dans lequel l'ensemble est traité avec une solution d'hydroxyde de sodium à une concentration comprise entre 0,5 % et 5 % pendant une période de 2 à 24 heures

9. Processus visant à obtenir une endoprothèse selon la revendication 2 ou 3, dans lequel les bactéries produisant de la cellulose sont des bactéries du groupe qui comprend les sous-espèces du genre Acetobacter xylinum, pasteurianus, rancens, ainsi que les Bacterium xylinoides.

10. Processus visant à obtenir une endoprothèse selon la revendication 9, dans lequel le milieu de culture (12) est inoculé avec la bactérie Acetobacter xylinum.

11. Processus visant à obtenir une endoprothèse selon la revendication 9 ou 10, dans lequel le milieu de culture (12) est approprié pour la production de cellulose biosynthétisée.

12. Endoprothèse, dans laquelle celle-ci est recouverte d'une membrane cellulosique biosynthétique (13) composée d'une structure de treillis métallique (10), avec une forme qui est essentiellement égale à la forme de l'endoprothèse, recouverte à l'extérieur d'une membrane cellulosique biosynthétique (13).

13. Endoprothèse selon la revendication 12, dans laquelle ladite structure (10) se compose d'un corps de treillis métallique en acier inoxydable qui possède une forme essentiellement cylindrique et est recouvert à l'extérieur d'une membrane (13) de cellulose biosynthétique.

14. Endoprothèse selon la revendication 13, dans laquelle ladite structure présente une face interne fortement polie et une surface externe brute.

15. Endoprothèse selon les revendications 12, 13 ou 14, dans laquelle ladite membrane (13) est imprégnée d'une substance thérapeutique.

16. Endoprothèse selon la revendication 12, dans laquelle ladite structure se compose d'un métal auto-expansible avec mémoire de son diamètre préconçu.

17. Endoprothèse selon les revendications 12 à 14, dans laquelle sont prévus des moyens externes de rétention (24, 26', 27',32) destinés à maintenir en place une membrane(23) de ce type.

18. Endoprothèse selon la revendication 17, dans laquelle des moyens externes de rétention de ce type consistent en une seconde maille expansible de forme (24) essentiellement cylindrique composée d'un matériau inerte flexible qui est juxtaposée à une membrane de ce type (23).

19. Endoprothèse selon la revendication 18, dans laquelle la proportion de matériau par rapport aux espaces vides dans la première (20) et la seconde (24) mailles, après expansion, est comprise entre 6 % et 8 %.

20. Endoprothèse selon la revendication 17, dans laquelle lesdits moyens externes de rétention se composent de canaux annulaires (27') au niveau des deux extrémités de la maille de stent (20), impliquant et emprisonnant les extrémités (28, 29) de la membrane cellulosique (23).

21. Endoprothèse selon la revendication 17, dans laquelle desdits moyens externes de rétention de ce type résulte d'une constriction radiale fournie par des bagues expansibles (32) placées sur les deux extrémités de la membrane cellulosique (23).

22. Processus visant à obtenir une endoprothèse selon la revendication 1, dans lequel l'endoprothèse est recouverte d'une membrane cellulosique biosynthétique (23) qui se compose d'une première maille expansible de forme (20) essentiellement cylindrique qui est composée d'un matériau inerte flexible, recouverte à l'extérieur par une membrane inégale (23) de forme essentiellement cylindrique, produite *in loco* par une culture de microorganismes synthétisant de la cellulose, comprenant les étapes consistant à :
insérer l'ensemble formé par une telle première structure (premier stent) (20) et ladite membrane (23), comprenant à l'intérieur un ballonnet dégonflé (25), dans une seconde structure (second stent) (24) de diamètre supérieur à celui de la première structure(20) ;
gonfler ledit ballonnet (25) et à dilater la première structure (premier stent) (20) et ladite membrane cellulosique (23), jusqu'à ce qu'il soit juxtaposé à l'intérieur à ladite seconde structure (24) (second stent), fournissant la rétention par la compression de ladite membrane cellulosique (23).

23. Processus visant à obtenir une endoprothèse selon la revendication 1, dans lequel l'endoprothèse est recouverte d'une membrane cellulosique biosynthétique (23) qui se compose d'une première maille expansible de forme (20) essentiellement cylindrique qui est composée d'un matériau inerte flexible, recouverte à l'extérieur par une membrane inégale (23) de forme essentiellement cylindrique, produite *in loco* par une culture de microorganismes synthétisant de la cellulose, comprenant les étapes consistant à river (31) les extrémités (26, 27) de ladite maille qui s'étend au-delà des extrémités (28, 29) de ladite membrane cellulosique (23) formant des canaux annulaires (27') qui encerclent les deux extrémités de ladite maille et entourent les extrémités (28, 29) de ladite membrane (24).
